# FASCICULE DE BREVET EUROPEEN

(11) **EP 2 182 964 B1**
(45) Date de publication et mention de la délivrance du brevet: **11.09.2013**
(21) Numéro de dépôt: 08834941.0
(22) Date de dépôt: 21.07.2008
(51) Int. Cl.: A61K 36/55, A61K 8/97, A61Q 19/08, A61Q 19/00, A61Q 17/04, A61P 17/16

(54) **UTLISATION D'UN EXTRAIT DE LIN DANS UNE COMPOSITION COSMÉTIQUE OU DERMATOLOGIQUE DESTINEE A ACTIVER LE CYTOCHROME C**
VERWENDUNG EINES FLACHSEXTRAKTS IN EINER KOSMETISCHER ODER DERMATOLOGISCHER ZUSAMMENSETZUNG ZUR AKTIVIERUNG VON CYTOCHROM C
USE OF A FLAX EXTRACT IN A COSMETIC OR DERMATOLOGICAL COMPOSITION FOR ACTIVATING CYTOCHROME C

(30) Priorité: 20.07.2007 FR 0705295
(43) Date de publication de la demande: 12.05.2010
(73) Titulaire: Société d'Extraction des Principes Actifs SA (Vincience), 06410 Biot (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2008/001072
(87) Numéro de publication internationale: WO 2009/043991

(56) Documents cités:
- WO-A-2004/010965
- WO-A-2004/057976
- WO-A-2008/015342
- US-A1- 2003 143 288

## Description

La présente invention se situe dans le domaine cosmétique et pharmaceutique, et plus particulièrement dans le domaine de la dermatologie. La présente invention concerne l'utilisation, dans une composition cosmétique d'une quantité efficace de principe actif issu du lin (genre Linum) ; pour activer le cytochrome c. De préférence, le principe actif provient de l'hydrolyse de protéines de lin et contient principalement des polypeptides ou des peptides. Il peut être utilisé seul ou en association avec au moins un autre principe actif. L'invention est également relative à l'utilisation d'une composition cosmétique pour stimuler les fonctions des mitochondries et à augmenter le niveau énergétique cellulaire. L'invention porte encore sur un procédé de traitement cosmétique destiné à protéger la peau et les phanères des agressions extérieures et à lutter contre le vieillissement cutané.

Le principe actif, activateur du cytochrome c, peut également être utilisé pour préparer des compositions pharmaceutiques destinées à prévenir ou lutter contre les pathologies liées à des dysfonctionnements mitochondriaux ; par exemple, certaines dégénérescences neuromusculaires ou cardiaques, le diabète de type II ou encore certaines pathologies du vieillissement.

Le terme « phanères » selon l'invention englobe l'ensemble des annexes kératiniques présentes à la surface du corps, en particulier les poils, les cils, les sourcils, les ongles et les cheveux.

La peau est un organe vital qui recouvre toute la surface du corps et assure des fonctions protectrices, sensitives, immunitaires, métaboliques ou encore thermorégulatrices. La peau, comme les autres organes, est soumise au vieillissement. Or, un des mécanismes majeurs impliqués dans les processus du vieillissement est l'accumulation de dommages oxydatifs dans des molécules essentielles telles que les lipides membranaires, les protéines, l'ADN et tout particulièrement l'ADN mitochondrial (ADNmt).

Les dommages oxydatifs sont provoqués par les radicaux libres, des espèces chimiquement instables et très réactionnelles générées par le métabolisme intracellulaire ou les agressions extérieures. Parmi ces agressions extérieures, on peut citer : les rayonnements UV, les toxines, les polluants atmosphériques, les oxydants alimentaires.

Dans la peau, on observe un vieillissement prématuré survenant dans les zones exposées aux rayonnements, caractérisé par des phénomènes d'altérations des macromolécules (péroxydation lipidique, carbonylation des protéines) touchant en particulier l'élastine, le collagène ou la fibronectine. On a également pu montrer un déclin progressif des fonctions mitochondriales avec l'âge, probablement lié à l'accumulation de mutations sur l'ADNmt (K. Singh, Ann. N.Y. Acad. Sci. 1019, 2004).

Une des conséquences importantes de l'accumulation des dommages oxydatifs est une réduction de la capacité de la cellule à produire de l'ATP (Porteous et al., Eur J Biochem 1998, 257(1):192-201). Ainsi, le phénomène du vieillissement cellulaire est en relation avec les dommages oxydatifs que subit la cellule mais aussi avec le processus de production d'énergie nécessaire à la cellule pour survivre.

L'organisme possède des mécanismes de défense capables de piéger ou de transformer les radicaux libres (enzymes, glutathion, vitamines A et E, coenzyme Q10, etc.). Cependant, ces systèmes de défenses antioxydants s'avèrent souvent insuffisants devant les nombreux stress et agressions extérieures auxquels sont soumis les organismes et la peau en particulier.

Dans ce contexte, les propriétés particulières du cytochrome c apparaissent comme particulièrement intéressantes :
Le cytochrome c est une petite protéine soluble de 15 kDa qui joue un rôle essentiel dans la fonction mitochondriale et dans la survie cellulaire. Le cytochrome c est une molécule hautement conservée chez la plupart des eucaryotes ; on la trouve dans les mitochondries des plantes, des animaux et de nombreux organismes unicellulaires. Le cytochrome c présente une structure protéique organisée autour d'une porphyrine, constituée de quatre noyaux pyrrol, eux-mêmes liés à un atome de fer.
Le rôle principal du cytochrome c est d'assurer le transfert d'électrons, grâce au changement de valence de l'atome de fer. Le cytochrome c qui est soluble, transporte ainsi les électrons depuis le complexe III (coenzyme QH2-cytochrome c réductase) jusqu'au complexe IV (cytochrome oxydase). Les électrons, qui sont le substrat de la cytochrome oxydase, sont alors transférés par l'enzyme vers l'oxygène.

La recherche de composés pouvant stimuler les mitochondries et augmenter le niveau énergétique cellulaire afin de prévenir ou de lutter contre les signes du vieillissement cutané, ou les dommages causés par les agressions extérieures, telles que les rayonnements UV, les radiations, ou les expositions à des toxines ou à des polluants, est une préoccupation importante de la recherche médicale et de la cosmétique. A cet égard, il a été proposé des solutions telles que l'apport de substances impliquées dans le métabolisme énergétique, et plus particulièrement d'intermédiaires ou de cofacteurs du cycle de Krebs tels que le fumarate, le L-malate, l'acétyl CoA (WO 02064129) ou encore le traitement de la peau par des substances capables de diminuer les radicaux libres, telles que la vitamine C (US 2004/0086526) ou la L-ergothionéine (WO 9836748). D'autre part des extraits de lignanes extraits du lin ont été décrit comme bénéfiques pour la peau (WO 2004/010965). D'autres extraits du lin ont été décrits comme ayant un effet bénéfique pour la peau. Un hydrolysat peptidique de lin a été décrit comme un stimulant de la synthèse de mélanine, pour protéger la peau et les phanères contre les agressions extérieures (WO2008/015342A), en particulier les UV. Des compositions topiques à base d'extraits de graines de lin et de réglisse ont également été décrites comme protégeant la peau contre les agents irritants tels que les rayonnements du soleil (US2003/143288A1). Enfin, le document WO2004/057976A décrit un procédé d'obtention de peptides inhibiteurs de l'ACE comprenant l'hydrolyse de protéines de graines ou de farine de lin. Cet extrait est destiné à être administré par voie orale, en tant qu'aliment ou en tant que médicament, pour traiter l'hypertension. Mais à la connaissance de la demanderesse, aucune composition cosmétique ou pharmaceutique comprenant des composés de nature peptidiques, issus du lin, capables d'activer le cytochrome c n'a encore été décrite.

La présente invention a pour principal objectif l'utilisation d'une quantité efficace d'un principe actif de nature peptidique, issu de l'hydrolyse des protéines du lin (genre Linum), dans une composition cosmétique pour activer le cytochrome c et stimuler les mitochondries, dans le but de protéger la peau des agressions extérieures et de lutter contre le vieillissement cutané. Ledit principe actif pourra être utilisé seul ou en association avec au moins un autre principe actif. Les inventeurs ont en effet mis en évidence une activité thérapeutique, et plus particulièrement dermatologique et cosmétique, d'un principe actif de nature peptidique issu de l'hydrolyse du lin, capable d'activer le cytochrome c. Il a notamment été mis en évidence que ces polypeptides ou ces peptides, qui constituent le principe actif, lorsqu'ils sont appliqués sur la peau, stimulent de façon importante les fonctions mitochondriales. Cela a, entre autre, été démontré par une augmentation de l'expression du cytochrome c, une augmentation de l'activité de la cytochrome oxydase et une augmentation de la synthèse d'ATP. Ce nouveau principe actif stimulateur des mitochondries, et plus généralement capable de protéger la peau des agressions extérieures permet ainsi d'ouvrir de nouvelles perspectives thérapeutiques et cosmétiques.

On entend par « principe actif capable de protéger la peau des agressions extérieures », un hydrolysat de nature peptidique issu de l'hydrolyse des protéines du lin (genre Linum), capable de présenter des propriétés protectrices ou de diminuer l'apoptose dans des cellules ou des tissus soumis à un stress d'origine physico-chimique ou environnementale.
On entend par « principe actif capable d'activer le cytochrome c », un hydrolysat de nature peptidique issu de l'hydrolyse des protéines du lin (genre Linum), capable d'augmenter l'expression du cytochrome c, soit par l'activation de la synthèse protéique (par modulation directe ou indirecte de l'expression génique du cytochrome c), soit par l'augmentation de l'activité biologique du cytochrome c, soit par d'autres processus biologiques tels que la stabilisation de la protéine cytochrome c ou encore la stabilisation des transcrits d'ARN messager.

On entend par « principe actif capable de stimuler les fonctions des mitochondries », toute un hydrolysat de nature peptidique issu de l'hydrolyse des protéines du lin (genre Linum), capable d'augmenter l'expression ou l'activité des principales molécules actives présentes dans les mitochondries, et plus généralement capables de d'augmenter les principales fonctions énergétiques de la mitochondries ; à savoir, la chaîne de phosphorylation oxydative et la synthèse d' ATP.
On entend par « de nature peptidique », un mélange de composés majoritairement représentés par des peptides ou des polypeptides.
Le terme « peptide » désigne un enchaînement de deux ou plusieurs acides aminés liés entre eux par des liaisons peptidiques ou par des liaisons peptidiques modifiées ; le terme « polypeptide » désignant un peptide de taille plus importante.

Par l'expression « biologiquement actif », on entend « qui possède une activité *in vivo* ou *in vitro* caractéristique de l'activité du principe actif selon l'invention ».

Le terme "hydrolysat ou issu de l'hydrolyse" désigne toute substance ou mélange de substances, ou préparation isolée, obtenue après hydrolyse de matière végétale.

Le principe actif selon l'invention peut être obtenu par extraction de protéines d'origine végétale, suivie d'une hydrolyse contrôlée qui libère des fragments peptidiques biologiquement actifs.

De très nombreuses protéines trouvées dans les plantes sont susceptibles de contenir des fragments peptidiques biologiquement actifs au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides biologiquement actifs selon l'invention, mais en s'assurant toutefois de la présence desdits fragments par des moyens analytiques appropriés.

Pour réaliser l'extraction, on peut utiliser la plante entière, ou une partie spécifique de la plante (feuille, graine, etc.).

Plus particulièrement selon l'invention, on utilise une des nombreuses plantes de la famille des linacées, du genre Linum (lin). Le genre Linum compte près de 200 espèces poussant surtout l'hémisphère nord. Ce sont des plantes herbacées à tiges fibreuses, à feuilles simples, aux fleurs à 5 pétales. Préférentiellement selon l'invention, on utilise l'espèce cultivée *Linum usitatissimu L.* Selon l'invention, le matériel végétal utilisé sera la graine et préférentiellement la graine débarrassée de son enveloppe par une étape de décorticage.

Dans une première étape, la plante est broyée à l'aide d'un broyeur à plantes. La poudre ainsi obtenue peut ultérieurement être "délipidée" à l'aide d'un solvant organique classique (comme par exemple un alcool, l'hexane ou de l'acétone).

On réalise ensuite l'extraction des protéines de la plante suivant le procédé classique (Osborne, 1924) modifié ; le broyat de plante est mis en suspension dans une solution alcaline contenant un produit adsorbant de type polyvinylpolypyrrolidone (PVPP) insoluble (0,01 - 20 %) ; en effet il a été observé que les opérations d'hydrolyses et de purifications ultérieures étaient facilitées par ce moyen. La concentration des substances de type phénoliques interagissant avec les protéines se trouve ainsi réduite.

La fraction soluble est recueillie après des étapes de centrifugation et de filtration, cette solution brute constituant alors une première forme de l'extrait contenant les protéines, les glucides et éventuellement des lipides.

Les protéines sont ensuite précipitées en faisant varier la force ionique en acidifiant le milieu, ce qui permet d'éliminer les composants solubles et les acides nucléiques.

Le précipité est ensuite lavé à l'aide d'un solvant organique tel que, par exemple, l'éthanol ou le méthanol puis le solvant est évaporé par séchage sous vide. Le précipité riche en protéines est remis en solution dans l'eau ou un autre solvant et constitue alors une forme plus purifiée de l'hydrolysat.

L'extraction peut également être réalisée en milieu neutre ou acide toujours en présence de polyvinylpolypyrrolidone. Après une étape de filtration, l'étape de précipitation s'effectue alors à l'aide d'un agent classique de précipitation tel que les sels (chlorure de sodium, sulfate d'ammonium) ou un solvant organique (alcool, acétone). Le précipité obtenu peut être séparé des agents de précipitation par dialyse après remise en solution dans de l'eau ou un autre solvant.

La fraction protéique isolée selon l'invention est ensuite hydrolysée dans des conditions ménagées pour générer des polypeptides et des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) et de micro-organismes (Aspergillus, Rhizopus, Bacillus, etc.).

Pour les mêmes raisons que précédemment, lors de cette étape d'hydrolyse ménagée, une quantité de polyvinylpolypyrrolidone est additionnée au milieu réactionnel. Après filtration la solution obtenue constitue l'hydrolysat actif. L'hydrolysat actif peut être encore purifié afin de sélectionner les poids moléculaires et la nature des peptides générés. Le fractionnement peut s'effectuer avantageusement par ultrafiltration et/ ou par une méthode de type chromatographique.

L'une quelconque des formes plus ou moins purifiées de l'hydrolysat est alors solubilisée dans de l'eau ou dans tout mélange contenant de l'eau, puis stérilisée par ultrafiltration.

L'hydrolysat végétal obtenu selon l'invention est analysé qualitativement et quantitativement pour ses caractéristiques physico-chimiques et sa teneur en composés de nature protéique et peptidique. On entend par composés de nature peptidique, les fragments de protéines, les peptides et les acides aminés libres présents dans le mélange. Les peptides, acides aminés et fragments de protéines sont dosés selon les techniques classiques, bien connues de l'homme du métier.

Ainsi, selon un mode de réalisation avantageux de l'invention, l'hydrolysat végétal actif a un pH compris entre 4 et 7, et préférentiellement, entre 5 et 6, un extrait sec titrant entre 1 et 8 g/l, et de manière préférée entre 2 et 5 g/l, sa teneur en composés de nature peptidique est comprise entre 0,1 et 5g/l, et préférentiellement entre 0,5 et 2 g/l et sa teneur en sucres est de 0,5 à 2,5 g/1.

Selon un mode de réalisation avantageux de l'invention, le principe actif selon l'invention est préalablement solubilisé dans un ou plusieurs solvants cosmétiquement ou pharmaceutiquement acceptables, classiquement utilisés par l'homme de métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

La composition utilisable selon l'invention peut en particulier consister en une composition pour soins capillaires, et notamment un shampooing, un après-shampooing, une lotion de mise en plis, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non suivie d'un rinçage, ou encore sous forme de shampooing.

Elle peut également se présenter sous forme de teinture ou de mascara à appliquer au pinceau ou au peigne, en particulier sur les cils, les sourcils ou les cheveux.

Il est bien entendu que le principe actif selon l'invention peut être utilisé seul ou bien en association avec au moins un autre principe actif, dans une composition cosmétique ou pour la préparation d'une composition pharmaceutique et/ou dermatologique.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, adaptés à une application sur la peau, les lèvres et/ou les phanères.

Ces compositions comprennent les excipients nécessaires à leur formulation, tels que solvants, épaississants, diluants, tensioactifs, anti-oxydants, colorants, conservateurs, parfums.

Avantageusement, les compositions utilisables selon l'invention contiennent en outre d'autres principes actifs destinés à favoriser son action. Parmi ces autres principes actifs, on peut citer les principes actifs ayant une action anti-radicalaire ou antioxydante, choisis parmi la vitamine C, la vitamine E, le coenzyme Q10 et les extraits polyphénoliques de plantes.

Par « principes actifs anti-radicalaires », on entend tout composé capable de piéger les radicaux libres. Ces principes actifs sont capables de bloquer les réactions en chaînes des radicaux libres avant les étapes ultimes de dégradation des constituants biologiques de la peau et ont de ce fait une activité antioxydante. Parmi ces autres principes actifs, on peut également citer les principes actifs stimulant les synthèses des macromolécules dermiques (laminine, fibronectine, collagène), par exemple le peptide de collagène commercialisé sous le nom « Collaxyl® » par la société Vincience.

Parmi ces autres principes actifs, on peut enfin citer les principes actifs stimulant le métabolisme énergétique, comme le principe actif commercialisé sous la dénomination « GP4G® » par la société Vincience.

Selon un autre aspect, la composition selon l'invention peut être une composition solaire, c'est-à-dire une composition aidant à la protection contre le rayonnement solaire. Ainsi, il peut être avantageusement ajouté, à la composition selon l'invention, des actifs aidant à la protection solaire tels que, par exemple, des filtres solaires.

Il est bien évident que l'invention s'adresse aux mammifères en général et plus particulièrement aux êtres humains.

La quantité efficace de principe actif correspond à la quantité nécessaire afin d'obtenir le résultat recherché, à savoir : activer le cytochrome c, stimuler les mitochondries et augmenter le niveau énergétique cellulaire, et plus généralement, protéger la peau et les phanères des agressions extérieures et lutter contre le vieillissement cutané

Selon un mode de réalisation avantageux de l'invention, le principe actif est présent dans les compositions de l'invention à une concentration comprise entre 0,0001 % à 20 % environ, et préférentiellement à une concentration comprise entre 0,05 % et 5 % environ par rapport au poids total de la composition finale.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Par ses activités particulières, le principe actif selon l'invention pourra être utilisé avantageusement dans une composition cosmétique ou pour la préparation d'une composition pharmaceutique.

En particulier, le principe actif selon l'invention pourra être utilisé avantageusement dans une composition cosmétique destinée à lutter de manière préventive et/ou curative contre les manifestations du vieillissement cutané et, plus spécifiquement, afin de lutter contre et/ou de prévenir le vieillissement photo-induit (photo-vieillissement). Par manifestations cutanées du vieillissement, on entend toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV). Le principe actif selon l'invention, ou la composition le contenant, permettra de lutter, en particulier, contre la perte d'élasticité et de fermeté de la peau.

Le principe actif selon l'invention permet de protéger la peau et les phanères contre tous types d'agressions extérieures. L'utilisation du principe actif, ou d'une composition le contenant, va permettre à la peau et aux phanères d'être protégés et de mieux résister aux stress environnementaux.

On entend, par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure », due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette.

Le principe actif selon l'invention peut être avantageusement utilisé dans une composition cosmétique ou pour la préparation d'une composition pharmaceutique, en tant qu'agent photo-protecteur et, plus particulièrement, en tant qu'agent photo-protecteur dit « secondaire ». On distingue, en effet, les agents photo-protecteurs primaires des agents photo-protecteurs secondaires. Les agents photo-protecteurs primaires sont des substances qui exercent un pouvoir physique : ils sont en mesure d'absorber les rayonnements UV et de les restituer sous forme de chaleur afin de protéger la peau. Les agents photo-protecteurs secondaires sont des substances qui ont généralement un effet biologique ; ce sont, par exemple, les agents capables de limiter les dommages causés à l'ADN et aux membranes par la pénétration des rayonnements UV dans la peau.

L'invention se rapporte encore à l'utilisation dans une composition cosmétique, ou pour la préparation d'une composition pharmaceutique, d'une quantité efficace de principe actif selon l'invention, le principe actif ou la composition le contenant, étant destiné à augmenter la synthèse d'ATP intracellulaire des cellules de la peau.

L'invention a également pour objet l'utilisation dans une composition cosmétique, ou pour la préparation d'une composition pharmaceutique, d'une quantité efficace de principe actif selon l'invention, le principe actif, ou la composition le contenant, étant destiné à prévenir les dommages causés à la peau par une exposition au soleil ou une exposition à des rayonnements ionisants lors de radiothérapies.

L'invention a également pour objet l'utilisation dans une composition cosmétique, ou pour la préparation d'une composition pharmaceutique, d'une quantité efficace de principe actif selon l'invention, le principe actif, ou la composition le contenant, étant destiné à stimuler les mitochondries, en particulier sur les zones du corps exposées aux rayonnements UV.

L'invention se rapporte encore à l'utilisation dans une composition cosmétique, ou pour la préparation d'une composition pharmaceutique, d'une quantité efficace de principe actif tel que décrit précédemment, le principe actif, ou la composition le contenant, étant destiné à protéger la peau des dommages causés par les radicaux libres.

L'invention consiste encore en l'utilisation d'une quantité efficace de principe actif pour préparer une composition pharmaceutique destinée à prévenir ou à lutter contre certaines pathologies liées à des dysfonctionnements mitochondriaux, par exemple, certaines dégénérescences neuromusculaires ou cardiaques, le diabète de type II, certaines pathologies du vieillissement.

L'invention consiste encore en un procédé de traitement cosmétique destiné à stimuler les défenses et à protéger la peau et les phanères des agressions extérieures et à lutter contre le vieillissement cutané, caractérisé par l'application sur la peau ou les phanères à traiter d'une composition contenant une quantité efficace de principe actif selon l'invention.

L'invention consiste encore en un procédé de traitement cosmétique destiné à donner un aspect plus sain à la peau et à améliorer l'éclat et la radiance du teint, caractérisé par l'application sur la peau à traiter d'une composition contenant une quantité efficace de principe actif selon l'invention.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Préparation de principe actif à partir de lin (Linum usitatissimum L.)

Le principe actif est obtenu à partir de plantes de l'espèce *Linum usitatissimum. L.* Bien entendu l'extrait peut être préparé à partir de plantes d'au moins l'une quelconque des nombreuses variétés et espèces appartenant au genre *Linum.*

Dans une première étape, 1 kg de graines de lin décortiquées sont broyées dans un broyeur à céréales. La farine obtenue est délipidée par l'action d'un solvant organique, l'hexane. Après filtration et séchage sous vide, la poudre obtenue est mise en suspension dans une solution aqueuse alcaline (dilution au 1/10) pH 10 contenant 1 % de polyvinylpolypyrrolidone (Polyclar V ISP). Ce mélange est maintenu sous agitation pendant un temps suffisamment long pour permettre la solubilisation des fractions solubles. La température d'extraction est variable (comprise entre 4 et 80°C) ; préférentiellement l'opération sera réalisée à froid. Après cette phase d'extraction le milieu est clarifié par centrifugation puis filtré sur filtre à plaque. Ce filtrat qui contient les fractions solubles du lin est ensuite soumis à une précipitation des protéines en faisant varier la force ionique en milieu neutre ou acide, ce qui permet d'éliminer les composants glucidiques solubles, les lipides et les acides nucléiques le milieu est amené à pH 3,5. Le surnageant est éliminé et le précipité est ensuite lavé à l'aide d'un solvant tel que, par exemple, l'éthanol ou le méthanol puis le solvant est évaporé par séchage sous vide.

A ce stade, on obtient environ 50 grammes de poudre de couleur jaune clair d'extrait protéique brut contenant :
- Protéines : 75 %
- Glucides : 20 %
- Lipides : 5 %

Le précipité riche en protéines est remis en solution dans l'eau ou un autre solvant.

L'extrait protéique brut est alors soumis à une série d'hydrolyses ménagées et sélectives consistant en des hydrolyses chimiques et enzymatiques en présence de 0,5 % de PVPP (Polyclar V) et d'endopeptidases à cystéine (papaïne, ficine). Après réaction l'hydrolysat est filtré sur plaque puis sur cartouche stérilisante (0,2 µm).

On obtient alors un hydrolysat de couleur claire, titrant de 15 à 30 g/l d'extrait sec, qui est alors dilué de telle sorte que la concentration en composés de nature peptidique déterminée par la méthode de Lowry, soit comprise entre 0,1 et 5g/l et préférentiellement entre 0,5 et 2 g/1. L'analyse physico-chimique de l'hydrolysat végétal, qui constitue le principe actif, montre que son pH est compris entre 4 et 7, et préférentiellement entre 5 et 6, l'extrait sec titre de 1 à 8 g/l et de manière préférée entre 2 et 5 g/l, sa teneur en composés de nature peptidique est comprise entre 0,1 et 5g/l, et préférentiellement, entre 0,5 à 2 g/l et sa teneur en sucres entre 0,5 à 2,5 g/1.

### Exemple 2 : Préparation de principe actif à partir de lin (Linum usitatissimum L.)

Une variante du protocole de l'exemple 1 consiste à réaliser la même séquence d'hydrolyses enzymatiques ménagées et sélectives mais en présence de 0,5 % de PVPP.

On obtient alors un hydrolysat de couleur claire titrant de 15 à 30 g/l d'extrait sec après filtration stérilisante

On procède ensuite à une ultrafiltration de la solution sur une cartouche de filtration Millipore Helicon (seuil de coupure ; 1 kDa). Les hauts poids moléculaires contenus dans le retentât sont écartés, le filtrat est conservé.

La concentration en composés de nature peptidique est déterminée par la méthode de Lowry, soit comprise entre 0,1 et 5g/l et préférentiellement entre 0,5 et 2 g/l. L'analyse physico-chimique de l'hydrolysat végétal, qui constitue le principe actif, montre que son pH est compris entre 4 et 7, et préférentiellement, entre 5 et 6, l'extrait sec titre entre 1 à 8 g/l, et de manière préférée entre 2 et 5 g/l, sa teneur en composés de nature peptidique est comprise entre 0,1 et 5g/l et préférentiellement entre 0,5 à 2 g/l et sa teneur en sucres entre 0,5 à 2,5 g/1.

Une autre variante consiste à effectuer une purification du principe actif, obtenu selon l'exemple 1 ou 2, par chromatographie d'échange d'ions, sur une colonne TSK gel (TosoHaas) avec un tampon phosphate pH 7.

### Exemple 3 : Mise en évidence de l'effet stimulant du principe actif selon l'exemple 1 sur la synthèse d'ATP intracellulaire

Le but de cette étude est de déterminer l'influence du principe actif selon l'exemple 1 sur la synthèse d'ATP, produit par les mitochondries.

Protocole : Cette étude s'effectue à l'aide d'un Kit « ATP Bioluminescence Assay Kit HS II » (Roche Applied Science). Des fibroblastes dermiques sont traités avec une solution à 1 %, de principe actif selon l'exemple 1, pendant une période allant de 1 à 3 heures. A la fin de l'incubation, les puits sont rincés avec 2 ml de PBS froid avant d'ajouter 250 µl d'un tampon de lyse fournis par le kit. Les cellules de chaque puits sont ensuite grattées puis récoltées dans des tubes de 14 ml. Chaque puits est rincé avec 2 x 500 µl PBS froid et le tout est de nouveau récolté dans les tubes respectifs. A partir de ces échantillons, une dilution est réalisée au 1/12000^{ième} dans du PBS froid avant chaque lecture. Le dosage d'ATP est réalisé sur ces échantillons : 50 µL de cette dilution sont déposés dans une luma-cuvette et 50 µL de luminol sont ajoutés. Après 10 secondes, la lecture de la luminescence est déclenchée. Les valeurs sont standardisées par rapport à la quantité de protéines pour chaque échantillon. Les mesures sont effectuées à l'aide d'un appareil : le Biocounter M2010A LUMAC®/3M.

Résultats : Les dosages d'ATP montrent qu'il y a une augmentation importante de la quantité d'ATP intracellulaire après 1 heure et 3 heures, dans des cellules traitées par le principe actif selon l'exemple 1 , en comparaison des cellules non traitées.

Conclusion : Le principe actif selon l'exemple 1 augmente sensiblement le niveau énergétique de cellules cutanées telles que les fibroblastes, et plus généralement stimule les fonctions mitochondriales.

### Exemple 4 : Mise en évidence de l'effet activateur du principe actif selon l'exemple 1 sur l'expression de cytochrome c

Le but de cette étude est de déterminer l'influence du principe actif selon l'exemple 1 sur l'expression du cytochrome c. Pour cela la quantité de cytochrome c a été évaluée par la technique de l'immuno-transfert (ou western blot).

Protocole : Des fibroblastes dermiques humains sont traités avec une solution à 1 %, de principe actif selon l'exemple 1, pendant 72 heures. Les cellules sont ensuite lysées et homogénéisées par sonication, puis centrifugées 10 minutes à 1000 g. Les échantillons, standardisés pour leur concentration en protéine (dosage kit BCA, Pierce), sont soumis à une électrophorèse sur gel Bis-TRIS 4-12 % (InvitroGen). Après électro-transfert, les membranes sont incubées pendant une nuit avec un anticorps anti-cytochrome c, dilué au 1/500 (Mouse monoclonal anti cytochrome c, TEBU). Un anticorps secondaire, couplé à la peroxydase et dilué au 1/5000 est ensuite utilisé (peroxydase conjugated F(ab')₂, Goat antimouse, Immunotech). Le signal chimie-luminescent est ensuite quantifié à l'aide du kit Supersignal West Femto Trial kit et lu dans une chambre de lecture (Multilmage light Cabinet, Alpha Immunotech Corporation).

Résultats : On observe une nette augmentation de l'expression du cytochrome c dans les fibroblastes traités par le principe actif selon l'exemple 1.

Conclusions : Le principe actif selon l'exemple 1 stimule fortement l'expression du cytochrome c dans les cellules cutanées, et plus généralement les fonctions mitochondriales.

### Exemple 5: Mise en évidence de l'effet activateur du principe actif selon l'exemple 1 sur l'activité enzymatique de la cytochrome oxydase

Le but de cette étude est de déterminer l'influence du principe actif selon l'exemple 1 sur l'activité mitochondriale. Pour cela l'activité enzymatique totale (mitochondriale) de la cytochrome oxydase a été mesurée.

Protocole : Des fibroblastes humains normaux sont traités avec une solution à 1 %, de principe actif selon l'exemple 1, pendant 3 heures et 24 heures. Les cellules sont récoltées, rincées puis lysées par sonication. Une première centrifugation est réalisé pour éliminer les principaux débris cellulaires et le surnageant est ensuite centrifugé de nouveau à 10 000 g pendant 10 min. L'activité enzymatique est dosée dans le surnageant et/ou dans le 2^{eme} culot, par une méthode biochimique, à l'aide de kit Cytocox 1 (Sigma) puis standardisée pour la teneur en protéines (dosée par le kit BCA, Pierce).

Résultats : Les dosages montrent qu'il y a une augmentation très importante de l'activité enzymatique de la cytochrome oxydase après 3 heures et 24 heures d'application du principe actif selon l'exemple 1, en comparaison des cellules non traitées.

Conclusion : Le principe actif selon l'exemple 1 stimule très fortement l'activité enzymatique de la cytochrome oxydase, et plus généralement l'activité mitochondriale, dans les cellules cutanées.

### Exemple 6 : Mise en évidence de l'effet protecteur du principe actif selon l'exemple 1 sur le potentiel de membrane mitochondriale

Le but de cette étude est de déterminer l'effet protecteur du principe actif selon l'exemple 1, vis-à-vis des mitochondries de fibroblastes dermiques soumis à un stress oxydatif, provoqué par de l'eau oxygénée (H₂O₂) ou à une irradiation UVB. On utilise pour cela un marqueur du potentiel de membrane des mitochondries (JC-1). Le JC-1 est un marqueur qui émet une fluorescence différente selon le niveau de polarisation de la membrane mitochondriale.

Protocole : Les fibroblastes dermiques humains sont traités avec une solution à 1 % de principe actif selon l'exemple 1, pendant 96 heures, puis soumis à un stress oxydatif, provoqué par de l'H₂O₂ à 2 mM pendant 30 minutes, ou à une irradiation par des UVB à 50 mJ/cm². Des contrôles non traités par le peptide ou par de l'H₂O₂ ou non irradiés sont réalisés dans les mêmes conditions. A la fin de l'expérimentation, les cellules sont lavées, fixées et soumises à un marquage par une solution de JC-1 (Molecular Probes) à 0,2 µg/ml, afin de révéler le potentiel de membrane des mitochondries.

Résultats : Dans les fibroblastes traités par le principe actif selon l'exemple 1, les mitochondries présentent une fluorescence rouge (JC-1 agrégé), signe d'un potentiel de membrane élevé, plus important que dans les cellules contrôle. Les fibroblastes irradiés ou soumis à un stress oxydatif ont des mitochondries qui fluorescent peu dans le rouge, et principalement dans le vert (JC-1 monomérique), signe d'une altération du potentiel de membrane mitochondrial. Dans ces dernières conditions, l'application du principe actif selon l'exemple 1 permet d'observer une fluorescence rouge plus marquée.

Conclusions : L'application du principe actif selon l'exemple 1 provoque une augmentation du potentiel de membrane des mitochondries. D'autre part, le principe actif selon l'exemple 1 protège efficacement les mitochondries des cellules cutanées soumises à un stress oxydatif ou à une irradiation par des UVB.

### Exemple 7 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |

| Noms commerciaux | Noms INCI | % massique |
|---|---|---|
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Principe actif de l'exemple 1 | | 3 |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2 -Lait après-soleil :

| Noms commerciaux | Noms INCI | % massique |
|---|---|---|
| PHASE A | | |
| Montanov L | C14-22 Alcohols (and) C12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| PHASE B | | |
|---|---|---|
| Eau déminéralisée | Aqua (Water) | qsp |

| PHASE C | | |
|---|---|---|
| Simulgel EG | Sodium Acrylate/Acryloyldimethyl Taurate | 0,4 |
| | Copolymer (and) Isohexadecane (and) Polysorbate 80 | |
| | Copolymer (and) Polysorbate 80 | |

| PHASE D | | |
|---|---|---|
| Phenonip | Phenoxyethanol (and) Methylparaben (and) Ethylparaben (and) Butylparaben (and) Propylparaben (and) Isobutylparaben | 0,30 |
| | Ethylparaben and Propylparaben and Buthylparaben | |

| Germall 115 | Imidazolidinyl Urea | 0,20 |
|---|---|---|
| PHASE E | | |
| Principe actif de l'exemple 1 | | 0,1 |

Préparer la phase A sous agitation. Incorporer la gomme xanthane progressivement, sous agitation défloculeuse. Les phases C et D seront incorporées une fois le gel terminé. La phase E, préparée préalablement jusqu'à parfaite dissolution de la DHA, sera rajoutée ensuite. Ajuster le pH si nécessaire à 4 - 4,5. Colorer et parfumer.

### 3 -Crème anti-âge :

| ***Noms commerciaux*** | ***Noms INCI*** | ***% massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Principe actif de l'exemple 1 | | 0,5 |

| ***Noms commerciaux*** | ***Noms INCI*** | ***% massique*** |
|---|---|---|
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors addi-tionnée si souhaité.

### 4 -Crème protectrice de jour :

| ***Noms commerciaux*** | ***Noms INCI*** | ***% massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Principe actif de l'exemple 1 | | 5 |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Hydrolysat de lin (genre Linum) activateur du cytochrome c, **caractérisé en ce qu'**il provient de l'hydrolyse des protéines du lin ;
- qu'il est de nature peptidique ;
- qu'il a un pH compris entre 4 et 7 ;
- un extrait sec titrant entre 1 et 8 g/l ;
- une tenseur en composés de nature peptidique comprise entre 0,1 et 5 g/l et ;
- une teneur en sucres est de 0,5 à 2,5 g/1.

2. Hydrolysate selon la revendication 1, **caractérisé en ce qu'**il est solubilisé dans un ou plusieurs solvants cosmétiquement ou pharmaceutiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

3. Hydrolysat selon l'une des revendications 1. ou 2, **caractérisé en ce qu'**il présente un pH compris entre 5 et 6.

4. Hydrolysat selon l'une des revendications précédentes, **caractérisé en ce qu'**il comprend un extrait sec titrant entre 2 et 5 g/l.

5. Hydrolysat selon l'une des revendications précédentes, **caractérisé en ce que** sa teneur en composés de nature peptidique est comprise entre 0,5 et 2 g/l.

6. Composition cosmétique, **caractérisée en ce qu'**elle comprend en tant que principe actif activateur du cytochrome c, un hydrolysat peptidique de lin (genre Linum) tel que défini dans l'une des revendications précédentes, utilisé seul ou en association avec au moins un autre principe actif.

7. Composition selon la revendication 6, **caractérisée en ce que** ledit principe actif est présent en une quantité représentant de 0,0001 % à 20 % du poids total de la composition, et préférentiellement en une quantité représentant de 0,05 % à 5 % du poids total de la composition.

8. Composition selon l'une quelconque des revendications précédentes, **caractérisée en ce qu'**elle contient en outre, au moins un principe actif antioxydant et/ou au moins un principe actif stimulant la synthèse de la matrice extracellulaire, et/ou au moins un principe actif stimulant le métabolisme cellulaire énergétique.

9. Utilisation cosmétique d'une quantité d'hydrolysat de lin selon l'une des revendications 1 à 5, pour protéger La peau et les phanères contre tous les types d'agressions extérieures.

10. Utilisation selon la revendication 9, pour prévenir ou traiter les signes cutanés du vieillissement et/ou du photo-vieillissement.

11. Utilisation selon la revendication 9, **caractérisée en ce que** Le principe actif provient de graines de lin de l'espèce *Linum usitatissimum L.*

12. Utilisation selon la revendication 9, **caractérisée en ce que** les agressions extérieures sont les rayonnements UV.

13. Procédé de traitement cosmétique destiné à stimuler les défenses et à protéger la peau et les phanères des agressions extérieures, **caractérisé en ce que** l'on applique topiquement sur la peau ou les phanères à traiter une composition telle que définie dans l'une des revendications 6 à 8.

14. Procédé de traitement cosmétique destiné à donner un aspect plus sain à la peau et à améliorer l'éclat et la radiance du teint, **caractérisé en ce que** l'on applique topiquement sur la peau à traiter une composition telle que définie dans l'une des revendications 6 à 8.

## Patentansprüche

1. Flachshydrolysat (Gattung Linum), Aktivator des Cytochroms C, **dadurch gekennzeichnet, dass** es aus der Hydrolyse der Proteine des Flachses stammt,
- dass es vom Typ Peptid ist;
- dass es einen pH-Wert zwischen 4 und 7 aufweist;
- einen Titer-Trockenextrakt zwischen 1 und 8 g/l
- einen Gehalt an Zusammensetzungen vom Typ Peptid zwischen 0,1 und 5 g/l, und
- einen Gehalt an Zuckern zwischen 0,5 und 2,5 g/l.

2. Hydrolysat nach Anspruch 1, **dadurch gekennzeichnet, dass** es in einem oder in mehreren kosmetisch oder pharmazeutisch akzeptablen Lösemitteln solubilisiert ist, wie z. B. Wasser, Glycerol, Ethanol, Propylenglycol, Butylenglycol, Dipropylenglycol, ethoxylierte oder propoxylierte Diglykole, zyklische Polyole, Vaselin, pflanzliches Öl oder jeder Mischung dieser Lösemittel.

3. Hydrolysat nach einem der Ansprüche 1 oder 2, **dadurch gekennzeichnet, dass** es einen pH-Wert zwischen 5 und 6 aufweist.

4. Hydrolysat nach einem der vorhergehenden Ansprüche, **dadurch gekennzeichnet, dass** es einen Titer-Trockenextrakt zwischen 2 und 5 g/l aufweist.

5. Hydrolysat nach einem der vorhergehenden Ansprüche, **dadurch gekennzeichnet, dass** sein Gehalt an Zusammensetzungen vom Typ Peptid zwischen 0,5 und 2 g/l liegt.

6. Kosmetische Zusammensetzung, **dadurch gekennzeichnet, dass** sie als aktivierender Wirkstoff des Cytochroms C ein Peptidhydrolysat von Flachs (Gattung Linum) umfasst, wie in einem der vorhergehenden Ansprüche definiert, alleine oder in Verbindung mit mindestens einem anderen Wirkstoff verwendet.

7. Zusammensetzung nach Anspruch 6, **dadurch gekennzeichnet, dass** der Wirkstoff in einer Menge anwesend ist, die 0,0001 % bis 20 % des Gesamtgewichts der Zusammensetzung darstellt, und vorzugsweise in einer Menge, die 0,05 % bis 5 % des Gesamtgewichts der Zusammensetzung darstellt.

8. Zusammensetzung nach einem der vorhergehenden Ansprüche, **dadurch gekennzeichnet, dass** sie außerdem mindestens ein Antioxidationswirkstoff und/oder mindesten einen Wirkstoff enthält, der die Synthese der extrazellulären Matrix stimuliert, und/oder mindestens einen Wirkstoff, der den energetischen Zellstoffwechsel stimuliert.

9. Kosmetische Verwendung einer Menge von Flachshydrolysat nach einem der Ansprüche 1 bis 5, um die Haut und die Hautanhangsgebilde gegen alle Arten von äußeren Aggressionen zu schützen.

10. Verwendung nach Anspruch 9, um den Hautanzeichen der Alterung und/oder der Lichtalterung der Haut vorzubeugen.

11. Verwendung nach Anspruch 9, **dadurch gekennzeichnet, dass** der Wirkstoff aus Flachssamen der Gattung *Linum usitatissinium L* stammt.

12. Verwendung nach Anspruch 9, **dadurch gekennzeichnet, dass** die äußeren Aggressionen UV-Strahlen sind.

13. Verfahren zur kosmetischen Behandlung, dazu ausgelegt, um die Abwehrkräfte zu stimulieren und die Haut und die Hautanhangsgebilde vor den äußeren Aggressionen zu schützen, **dadurch gekennzeichnet, dass** topisch auf die zu behandelnde(n) Haut oder Hautanhangsgebilde eine Zusammensetzung aufgetragen wird, wie in einem der Ansprüche 6 bis 8 definiert.

14. Verfahren zur kosmetischen Behandlung, das dazu ausgelegt ist, um der Haut ein gesünderes Aussehen zu geben und den Glanz und die Leuchtkraft des Teints zu verbessern, **dadurch gekennzeichnet, dass** topisch auf die zu behandelnde Haut eine Zusammensetzung aufgetragen wird, wie in einem der Ansprüche 6 bis 8 definiert.

## Claims

1. Cytochrome c activating flax (Linum genus) hydrolysate, **characterised in that** it is obtained from flax protein hydrolysis;
- it is peptide in nature;
- it has a pH between 4 and 7;
- a dry extract containing between 1 and 8 g/l;
- a peptide compound content between 0.1 and 5 g/l and;
- the sugar content of 0.5 to 2.5 g/l.

2. Hydrolysate according to claim 1, **characterised in that** it is solubilised in one or a plurality of cosmetically or pharmaceutically acceptable solvents, such as water, glycerol, ethanol, propylene glycol, butylene glycol, dipropylene glycol, diglycol ethoxylate or propoxylate, cyclic polyols, petroleum jelly, a vegetable oil or any mixture of these solvents.

3. Hydrolysate according to any of claims 1 or 2, **characterised in that** it has a pH between 5 and 6.

4. Hydrolysate according to any of the above claims, **characterised in that** it comprises a dry extract containing between 2 and 5 g/l.

5. Hydrolysate according to any of the above claims, **characterised in that** the peptide compound content thereof is between 0.5 and 2 g/l.

6. Cosmetic composition, **characterised in that** it comprises, as the active substance for activating cytochrome c, a flax (Linum genus) peptide hydrolysate as defined in any of the above claims, used alone or in association with at least one other active substance.

7. Composition according to claim 6, **characterised in that** said active substance is present in a quantity representing 0.0001% to 20% of the total weight of the composition, and preferentially in a quantity representing 0.05% to 5% of the total weight of the composition.

8. Composition according to any of the above claims, **characterised in that** it further contains at least one antioxidant active substance and/or at least active substance for stimulating extracellular matrix synthesis, and/or at least one active substance for stimulating the cell energy metabolism.

9. Cosmetic use of a quantity of flax hydrolysate according to any of claims 1 to 5, for protecting the skin and skin appendages against all types of external attacks.

10. Use according to claim 9, for preventing or treating skin signs of ageing and/or photo-ageing.

11. Use according to claim 9, **characterised in that** the active substance is obtained from *Linum usitatissimum L.* species flaxseeds.

12. Use according to claim 9, **characterised in that** the external attacks are UV rays.

13. Cosmetic treatment method for stimulating the defences and protecting the skin and skin appendages from external attacks, **characterised in that** a composition as defined in any of claims 6 to 8 is applied topically to the skin or skin appendages.

14. Cosmetic treatment method for giving the skin a healthier appearance and enhancing the freshness and radiance of the complexion, **characterised in that** a composition as defined in any of claims 6 to 8 is applied topically to the skin to be treated.
